# EUROPEAN PATENT APPLICATION

(11) **EP 1 520 567 A1**
(43) Date of publication of application: **06.04.2005**
(21) Application number: 04023370.2
(22) Date of filing: 30.09.2004
(51) Int. Cl.: A61F 5/455, A61F 5/44

(54) **Automatic urine disposal device**

(30) Priority: 30.09.2003 JP 2003340487
(71) Applicant: Hitachi, Ltd., Tokyo (JP); Uni-Charm Corporation, Shikokuchuo-shi, Ehime 799-0111 (JP)
(72) Inventor: Tazoe, Wataru, Tsuchiura-shi Ibaraki 300-0022 (JP); Kobayashi, Junichi, Ushiku-shi Ibaraki 300-1237 (JP); Machida, Shigeru, Nishiibaraki-gun Ibaraki 319-0208 (JP); Miyagawa, Ryousuke, Kasukabe-shi Saitama 344-0032 (JP); Ishitsuka, Yoshikazu, Higashiibaraki-gun Ibaraki 319-0104 (JP); Wada, Ichiro, c/o Uni-Charm Corporation, Techn. C., Mitoyo-gun Kagawa 769-1602 (JP); Suzuki, Miou, c/o Uni-Charm Corporation, Techn. C., Mitoyo-gun Kagawa 769-1602 (JP)
(74) Representative: Beetz & Partner Patentanwälte

(57) **Abstract**

An automatic urine disposal device has a urine receptacle for suctioning urine discharged by a device wearer. A urine drainage port is formed on this urine receptacle, and from this urine drainage port, urine is directed into a urine tank (30). The urine tank is sealed by a lid, and tube guide holes (31 a, 31 b) are formed on the lid. The urine drainage tube (13) is connected to one of the tube guide holes via a mounting fixture (16) while maintaining the airtightness, and the urine drainage tube (13) can be detachable from/attachable to the tube guide hole.

## Description

The present application claims priority from Japanese application JP2003-340487 filed on September 30, 2003, the content of which is hereby incorporated by reference into this application.

### BACKGROUND OF THE INVENTION

The present invention relates to an automatic urine disposal device worn by the elderly with difficulty walking, hospitalized patients, physically disabled people, and others who are unable to voluntarily control the bladder or to clean up urine on their own.

In general, elderly with difficulty walking, physically disabled people, and those who are hospitalized due to injuries or illness sometimes become unable to voluntarily control the bladder or clean up urine on their own. In order to handle urination of those listed above, an automatic urine disposal device, which uses a vacuum pump to suction urine absorbed by a urine absorbent material and discharges the aforementioned urine into a urine tank, has been developed. The vacuum pump sucks air in a sealed urine tank to create a pressure difference between the inside of the tank and the atmosphere in order to draw urine, absorbed by the urine receptacle, into the urine tank through a urine drainage tube. Automatic urine disposal devices of such configuration have been disclosed in Japanese Application Patent Laid-open Publications No. Hei 07-171182 and No. 2003-126242.

An automatic urine disposal device proposed prior to the present invention sucks urine while a urine receptacle is touching a wearer's urinating part, or in other words, while the urine absorbent material of the urine receptacle is exposed to the atmosphere, and this causes lowering of the percentage of urine collection from the urine receptacle (urine absorbent material). This, in turn, causes an increase in the amount of residual urine in the urine receptacle (urine absorbent material), resulting in great discomfort for the wearer. The capacity of the vacuum pump must be made higher in order to reduce the amount of residual urine in the urine receptacle; however, this means that the automatic urine disposal device must be made heavier and larger. For this reason, the automatic urine disposal device proposed prior to the present invention has not been considered practical as a portable urine disposal device.

As an aside, in order to create negative pressure inside a urine tank so that urine will be drawn into the urine tank, a urine drainage tube must be connected to the urine tank. Conventionally, for example, a joint is attached to the tip of a guide tube of the urine tank so that a urine drainage tube can be inserted and connected to the guide tube via the joint. Such configuration has been disclosed in the aforementioned Japanese Application Patent Laid-open Publication No. 2003-126242.

In the prior art described above, a guide tube is provided on the urine tank to be connected to the urine drainage tube. When replacing the urine drainage tube, it is necessary to directly handle the parts in contact with urine and thus it tends to be unclean. Also, the urine drainage tube is tightly and deeply inserted in order to prevent urine leakage; therefore, it is troublesome when removing/inserting it. Especially, a portable automatic urine disposal device requires replacement of the urine drainage tube every day or every other day, and it is desirable that the tube replacement interval be made longer when making the device fit for practical use.

### BRIEF SUMMARY OF THE INVENTION

The objective of the present invention is to provide an automatic urine disposal device which allows easy and clean replacement of urine drainage tubes.

To achieve the aforementioned objective, the present invention is characterized in that a tube guide hole is formed on the lid that seals the urine tank such that a urine drainage tube may be inserted into the hole, and the urine drainage tube is connected to the urine tank by a mounting fixture that fixes the urine drainage tube to the tube hold while maintaining the airtightness of the urine tank.

Furthermore, the present invention is also characterized in that a tube guide hole is formed on the top surface of a sealed urine pack contained in the urine tank such that a urine drainage tube may be inserted into the hole while being held by the hole, and the urine drainage tube is connected to the urine pack by a mounting fixture that fixes the urine drainage tube to the tube guide hole while maintaining the airtightness of the urine pack.

The preferred shape of the mounting fixture is a funnel shape with a hole for inserting and holding the urine drainage tube, and also it is desirable that the fixture be made of flexible material and be attachable to/detachable from the tube guide hole.

### BRIEF DESCRIPTION OF THE SEVERAL VIEWS OF THE DRAWINGS

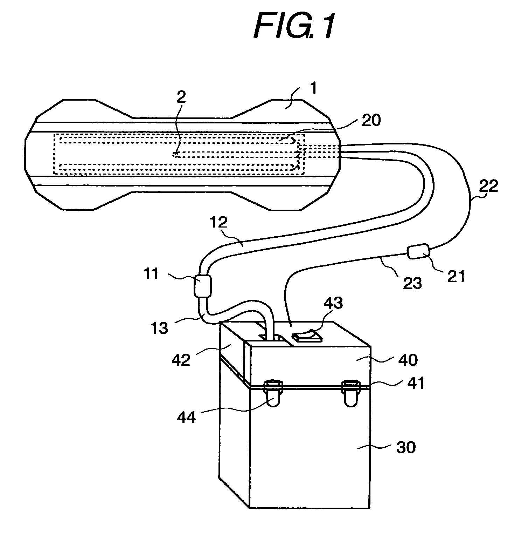

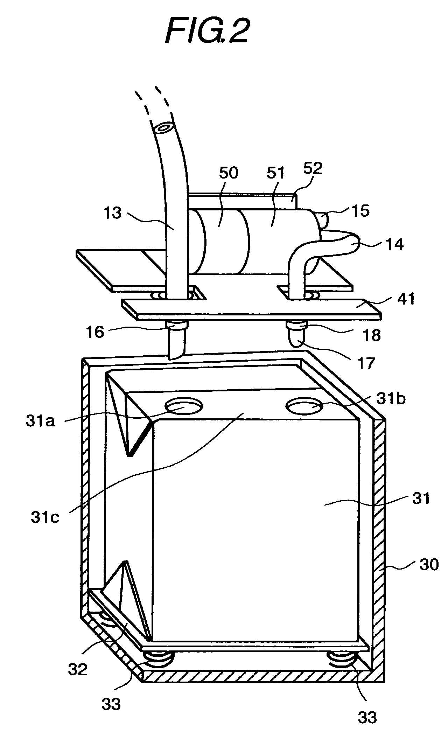

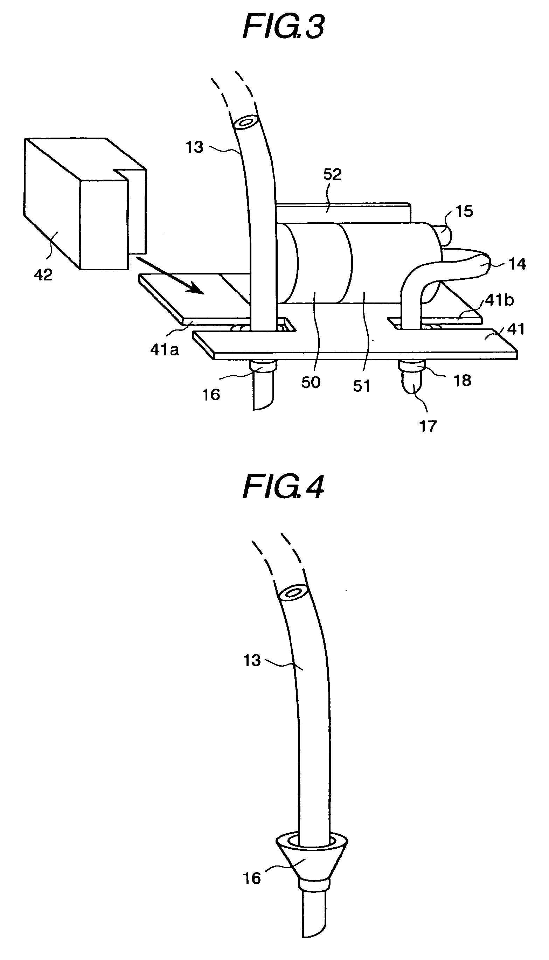

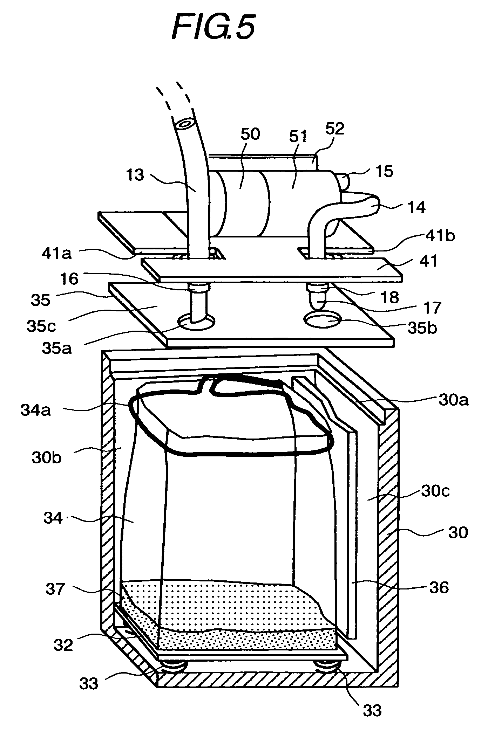

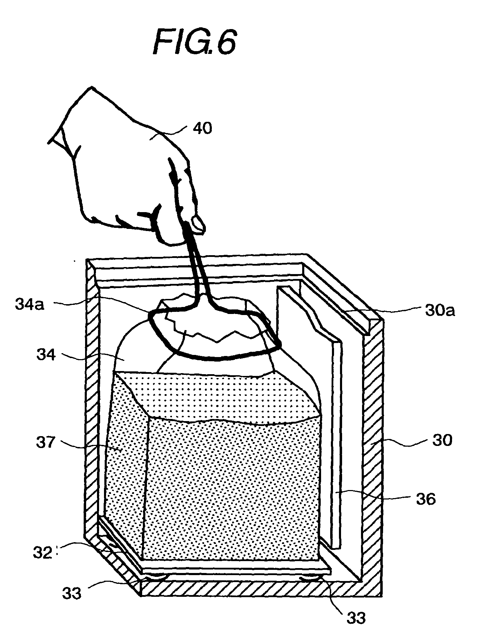

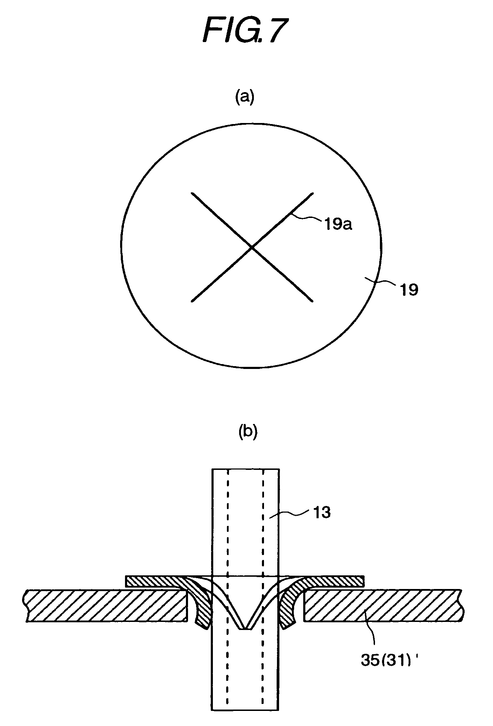

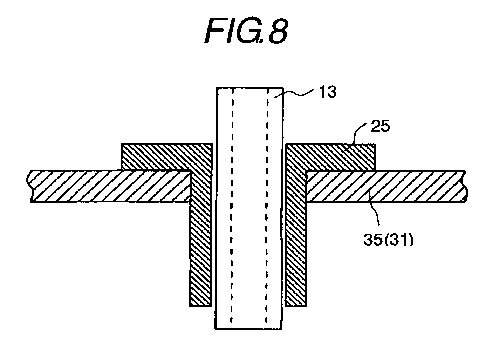

Figures 1 through 4 are diagrams showing an embodiment of an automatic urine disposal device of the present invention. Figure 1 is a perspective view of the entire device, Figure 2 is an exploded perspective view of the device with its urine tank partially removed and its lid case removed, Figure 3 is a perspective view of the lid plate with the lid case of the urine tank removed, and Figure 4 is a diagram showing a urine drainage tube with a mounting fixture attached. Figures 5 and 6 show another embodiment of the present invention, and Figures 7 and 8, to be referred to with reference to Figure 2, show vertical cross-sectional views of a mounting fixture applicable in such an embodiment.

### DETAILED DESCRIPTION OF THE INVENTION

Urine of a device wearer is absorbed by a urine receptacle. A urine tank is sealed by a lid with tube guide holes, and its bottom plate provides elastic suspension to the support plate. A urine pack with a thread that opens and closes the pack is placed on the support plate and contained in the urine tank. A urine drainage tube is designed to draw urine to the urine pack from a urine drainage port, formed on the urine receptacle, through the tube guide hole. The urine pack contains superabsorbent polymers that absorb urine and become gelatinized. A vacuum pump lowers pressure inside the urine tank to create negative pressure in the urine drainage port of the urine receptacle in order to suck urine into the urine tank to store it in the urine pack. A mounting fixture connects the urine drainage tube to the urine tank by fixing the urine drainage tube to the tube guide hold while maintaining the airtightness of the urine tank. The mounting fixture is made of flexible material and has a funnel-like shape, and is designed to be attachable to/detachable from the tube guide hold on the urine tank lid.

Figures 1 and 2 show an embodiment of the present invention. Figure 1 is a schematic diagram of the entire device, and Figure 2 is an exploded perspective view of the device with its urine tank partially removed and its lid case removed. Figures 1 and 2 show an example in which a urine pack as a urine storage container is placed inside the urine tank.

In Figure 1, a urine receptacle 1 that absorbs urine discharged from the urinating part of a device wearer (not shown) roughly has the shape of a rectangle, and its width at the middle in the longitudinal direction (direction of the wearer's front and rear) is narrower at the middle part. The urine receptacle is thus shaped like an hourglass so that it fits the wearer's crotch.

The urine receptacle 1 used in this present invention is the one that is able to improve urine collection efficiency, reduce the amount of residual urine, and allow vacuuming of urine by a small-capacity vacuum pump. On the urine receptacle 1, a urine drainage port 2 is formed. Also on the urine receptacle, a urine sensor 20 is formed for urination detection. The urine sensor 20 is an electric conductor and detects the wearer's urination based on change in resistance resulting from electric conduction triggered by urination.

One of the end points of a urine drainage tube 12 is bonded to the urine drainage port 2 formed on the urine receptacle 1. The other end of the urine drainage tube 12 is connected to a one-touch joint 11. The one-touch joint 11 is fixed to one of the end points of a urine drainage tube 12, and thus the one-touch joint 11 connects one end of the urine drainage tube 12 to one end of the urine drainage tube 13. The urine drainage tubes 12 and 13 are formed by soft, flexible materials mad e of soft resins. The one-touch joint 11 is made of soft materials.

A urine detection signal detected by the urine sensor 20 is inputted to a control board 52 via signal lines 22 and 23. The control board 52 controls a vacuum pump 51. The signal lines 22 and 23 are connected to each other by a one-touch joint 21.

A urine tank 30 is made of resins and has a shape of a rectangular solid with an opening on the top. A spring 33 is located at each corner of the bottom plate of the urine tank 30 to provide elastic suspension to a support board 32. A urine pack 31 as a urine storage container is sealed with a sheet of heavy paper like that of a milk carton, and two tube guide holes 31a and 31b are formed on a top surface 31c of the urine pack 31. Although not shown, the urine pack 31 contains particulate superabsorbent polymers such as cross-link polyacrylic acid soda that become gelatinized after absorbing urine. The superabsorbent polymers provide a urine swaying motion control means that prohibits urine from swaying (rippling) inside the urine pack 31.

The urine pack 31 is made with, for example, a sheet of heavy paper like that of a milk carton, and is shaped like a rectangular solid. A partition plate (not shown) is placed inside the pack so that the pack will not cave in when vacuumed. Capacity of the urine pack 31 is approximately 500 ml, which is sufficient for storing two instances of urination. As a portable device, however, it is desirable that its dimensions be adjusted to 110 mm × 110 mm × 50 mm (605 ml). Also, for long time use, such as clinical use or nighttime use, it is desirable that the capacity be made 1000 ml or more. Note that the shape of the urine tank 30 and the urine pack 31 can be changed in order to fit them to the shape of the wearer's body.

A lid plate 41 is located on the top opening of the urine tank 30, and a lid case 40 is provided so as to cover the lid plate 41. In the space defined by the lid case 40 and lid plate 41, a vacuum pump 51 is placed. The vacuum pump 51 is driven by a motor 50. One end of a suction tube 14 and an air release tube 15 are linked to the vacuum pump 51. Just as with the urine drainage tubes 12 and 13, the suction tube 14 and the air release tube 15 are formed by soft, flexible materials.

The motor 50 uses the battery placed inside a battery box 42, shown in Figure 3, as the driving power and is controlled by the controller mounted on the control board 52. The battery box 42 is installed in the lid case 40 like a cassette to supply power to the motor 50, and uses alkaline cells or secondary batteries. As for the size of the vacuum pump 51, the diameter is approximately 30 mm or smaller, the length is approximately 70 mm or shorter, and the battery voltage is 5 to 9 V.

On the top surface of the lid case 40, a manual switch 43 for manually driving the motor 50 is provided. A one-touch lock mechanism 44 is to be used when fixing the lid case 40 to the urine tank 30. On the lid plate 41, a cutout section 41a for guiding the urine drainage tube 13 and another cutout section 41b for guiding the suction tube 14 are formed. Both the urine drainage tube 13 and the suction tube 14 have a diameter of approximately 5 mm, and the width of each of the cutout sections 41a and 41b is approximately 6 mm.

On the other end of the urine drainage tube 13, a mounting fixture 16 is attached for fixing the urine drainage tube 13 to the urine pack 31. As seen in Figure 4, an insertion hole is formed in the mounting fixture 16 for inserting and holding the urine drainage tube 13, and the mounting fixture 16 fixes the urine drainage tube 13 to the tube guide hole 31a to connect the urine drainage tube 13 to the urine pack 31 while maintaining the airtightness of the urine pack 31. The mounting fixture 16 has a funnel-like shape and is made of flexible materials made of soft resins, and can be detached from/attached to the tube guide hole 31a.

The mounting fixture has its outer surface and inner surface in the shape of a taper. For example, the mounting fixture 16 has a taper angle of 30 degrees from its axis, the largest diameter of the outer surface taper is 8.0 mm, and the smallest diameter is approximately 6.1 mm. As mentioned earlier, the width of the cutout section 41a on the lid plate 41 is approximately 6 mm. When the urine drainage tube 13 is guided into the cutout section 41a, the tube 13 cannot be pulled upwards due to the mounting fixture 16, as seen in Figure 3.

The mounting fixture 16 is inserted into the tube guide hole 31a formed on the top surface 31c of the urine pack 31 from the side with the smallest diameter (6.1 mm), while holding the inserted urine drainage tube 13. The diameter of the tube guide hole 31a is approximately 6.5 mm, which is somewhere between the largest and smallest diameters of the taper provided on the outer surface of the mounting fixture 16. When the mounting fixture 16 is fixed to the tube guide hole 31a, the urine pack 31 becomes sealed while maintaining its airtightness. The mounting fixture 16 is fitted to the tube guide hole 31a by insertion, and is configured to be detachable from/attachable to the tube guide hole 31a. The urine drainage tube 13 becomes connected to the urine pack 31 by the mounting fixture 16 becoming fitted to the tube guide hole 31a.

The suction tube 14 has one end linked to the vacuum pump 51, and a filter 17 is attached on the other end. The filter 17 serves to remove the odor, but it also prevents fluid that may spread in the urine pack 31 at the time of urine suction from being vacuumed into the vacu um pump 51. A mounting fixture 18 is attached to that other side of the suction tube 14 to fix the suction tube 14 to the urine pack 31. This mounting fixture 18 is the same one as the mounting fixture 16 attached to the urine drainage tube 13. The mounting fixture 18 is fitted to the tube guide hole 31b by insertion, and is configured to be detachable from/attachable to the tube guide hole 31b. The mounting fixture 18 becomes fitted to the tube guide hole 31b while maintaining the airtightness of the urine pack 31.

Figures 2 and 3 show the urine drainage tube 13 with the mounting fixture 16 attached and the suction tube 14 with the mounting fixture 18, guided and fitted into the cutout sections 41a and 41b respectively. While maintaining the tubes and the mounting fixtures in that state, the lid plate 41 and then the lid case 40 are placed on the top opening of the urine tank 30 as shown in Figure 1. Ends of the urine drainage tube 13 and suction tube 14 are inserted into the tube guide holes 31a and 31b provided on the urine pack 31. The mounting fixtures 16 and 18 are pressed down below the cutout sections 41a and 41b provided on the lid plate 41, and the taper parts of the fixtures are inserted and glued to the tube guide holes 31a and 31b provided on the urine pack 31.

Meanwhile, the urine pack 31 is pressed upward by the support plate 32 elastically supported by the springs 33. This mechanism allows an increase in degree of adhesion between the mounting fixtures 16 and 18, and the tube guide holes 31a and 31b provided on the urine pack 31. Note that, application of a soft material on the surface 31c of the urine pack 31 having the tube guide holes 31a and 31b can also improve the degree of adhesion between the mounting fixtures 16 and 18, and the tube guide holes 31a and 31b.

In this configuration, the urine receptacle 1 is attached to the wearer (not shown) such that it touches his/her urinating part inside the underwear. The lid case 40 containing the urine tank 30, vacuum pump 51, and motor 50 will be carried around by the wearer, or is placed on or under the bed when the wearer is lying on a bed.

When the wearer urinates with the lid case placed in the manner described above, the urine sensor 20 on the urine receptacle 1 becomes electrically conductive, and sends a urine detection signal to the control board 52. The controller mounted on the control board 52 activates the motor 50 and then drives the vacuum pump 51. When the vacuum pump 51 is activated, air pressure inside the urine pack 31 becomes lower due to air release, creating negative pressure in the urine drainage port 2 of the urine receptacle 1. When negative pressure is created in the urine drainage port 2, urine absorbed by the urine receptacle 1 is efficiently vacuumed by the urine drainage tube 12 due to suction power generated by negative pressure. Urine, vacuumed by the urine drainage tube 12, is then drawn by negative pressure into the urine pack 31 via the urine drainage tube 13 and is then stored therein. Note that, upon urinating, the wearer can use the manual switch 43 to activate the motor 50 to draw the urine into the urine pack 31.

Urine, drawn into the urine pack 31, then has chemical reactions with particulate superabsorbent polymers (not shown) and becomes gelatinized. Gelatinization of the urine can prevent the swaying (rippling) motion of the urine inside the urine pack 31. Therefore, it is possible to configure a portable automatic urine disposal device in a preferred form, in which urine will not leak even if it is carried around.

When urination by the wearer finishes and the urine detection signal from the urine sensor 20 is no longer sent out, the controller mounted on the control board 52 stops the motor 50 and then stops the vacuum pump 51. It is also possible to stop the motor and vacuum pump by using the manual switch 43. If the urine pack 31 has sufficient capacity for holding two-times-worth of urine, the next urination process is conducted in the same manner.

After urine is stored in the urine pack 31, the urine pack 31 must be discarded by first unlocking the lock mechanism 44 to separate the urine tank 30 and the lid case 40, and then by removing the mounting fixtures 16 and 18 from the urine pack 31. The urine pack 31 is made of paper and thus can be disposed of as burnable waste.

After using the urine receptacle 1 for a day or if it becomes dirty due to defecation, the urine receptacle 1 must be discarded and replaced with a new urine receptacle 1 by separating the urine drainage tubes 12 and 13 from the one-touch joint 11 and then by disconnecting the signal lines 22 and 23 from the one-touch joint 21. The urine receptacle can be disposed of as burnable waste.

From a hygiene standpoint, the urine drainage tube 13 must be replaced after one or two days of use. Replacement of the urine drainage tube 13 can be done by first disconnecting the urine drainage tubes 12 and 13 from the one-touch joint 11 and then by disconnecting the signal lines 22 and 23 from the one-touch joint 21. Then, after separating the urine tank 30 and the lid case 40 by unlocking the lock mechanism 44, the mounting fixture 16 must be removed from the tube guide hole 31a on the urine pack 31 as shown in Figure 2. The mounting fixture 16 can be easily removed by grabbing the mounting fixture 16 and pulling it out from the tube guide hole 31a. Replacement of the urine drainage tube 13 can be done easily as well as cleanly.

The drainage tube 13 must then be removed from the cutout section 41a and disposed of with the mounting fixture 16, followed by installation of a new urine drainage tube 13 into the tube guide hole 31a provided on the urine pack 31. Note that air leakage of the suction tube 14 can be examined in the same manner as replacement of the urine drainage tube 13.

Urine of the wearer is treated in the aforementioned manner. The urine drainage tube can be easily and cleanly replaced since the mounting fixture, with a urine drainage tube inserted, fixes the urine drainage tube to the tube guide hole while maintaining the airtightness of the urine pack and connects the urine drainage tube to the urine tank. This structure is significantly effective when used as a portable automatic urine drainage disposal device.

Also, insertion/removal of the mounting fixture into/from th e tube guide hole is particularly easy since the fixture has a funnel-like shape, or a taper shape, on the outer surface. Furthermore, the urine drainage tube can be easily inserted into the insertion hole since the inner surface also as a taper shape.

Figure 5 shows another embodiment of the present invention. This embodiment is different from the embodiment described above in that tube guide holes 35a and 35b are formed on a lid plate 35, and the lid plate 35 covers the top opening of the urine tank 30, and that the urine drainage tube 13 and the suction tube 14 are fixed in the tube guide holes 35a and 35b. The mounting fixture 16 for the urine drainage tube 13 is fit together with the tube guide hole 35a by insertion, and the mounting fixture 18 for the suction tube 14 is fit together with the tube guide hole 35b by insertion.

A groove 30a, for placing the lid plate 35 in, is formed on the top opening of the urine tank 30. Also, inside the urine tank 30, an area 30b in which the urine drainage tube 13 is placed and an area 30c in which the suction tube 14 is placed are separated by a partition plate 36, with space provided on the top and bottom. A urine bag 34 serving as a urine storage container is contained in the area 30b.

The urine bag 34 is made of a material such as polyethylene resin and has a square-shaped bottom. A thread 34a is provided at the opening of the urine bag 34 for opening and closing the bag. The thread 34a is locked by a plurality of hooks (not shown) provided on the inner wall surfaces of the urine tank 30. The urine bag 34 is kept open and is fixed such that it will not be dislocated in the urine tank 30. Superabsorbent polymers 37 are contained in the urine bag 34.

When the mounting fixtures 16 and 18 are attached to the tube guide holes 35a and 35b respectively, the urine tank 30 can be sealed with high airtightness. Therefore, in the second embodiment of the present invention as shown in Figure 5, urine discharged into the urine receptacle 1 will be suctioned and stored in the urine bag 34 by creating negative pressure in the urine tank 30. Urine, drawn into the urine bag 34, then has chemical reactions with superabsorbent polymers 37 and becomes gelatinized.

After storing urine in the urine bag 34, the top opening of the urine bag 34 can be closed like a purse by using a hand 40 to pull the thread 34a upward such that the thread 34a comes off the hooks. It is thus possible to dispose of the urine bag 34 without making the hand 40 dirty.

In the second embodiment of the present invention, too, the urine drainage tube can be easily and cleanly replaced since the mounting fixture, with a urine drainage tube inserted, fixes the urine drainage tube to the tube guide hole while maintaining the airtightness of the urine bag and connects the urine drainage tube to the urine tank. The urine bag as a urine storage container can be cleanly disposed of without making the hands dirty. Note that, in the second embodiment of the present invention as shown in Figure 5, it is clear that the suction tube can be firmly fixed and linked to the urine tank.

Figure 7 and 8 show other examples of mounting fixtures. In Figure 7, cross-shaped cracks 19a are made on the center of a disk-like plate 19 made of a soft material as shown in Figure 7(a), and then the urine drainage tube 13 is inserted into the cracks 19a as shown in Figure 7(b). The cracks 19a serve as an insertion hole for inserting and holding the urine drainage tube.

With such a mounting fixture 19, it is possible to connect the urine drainage tube 13 to the urine tank 30 or the urine pack 31 by fixing the urine drainage tube 13 to the tube guide hole while maintaining the airtightness of the urine tank 30 or the urine pack 31. Therefore, replacement of the urine drainage tube can be carried out easily as well as cleanly.

Figure 8 shows a mounting fixture 25 with a T-shape cross section. With such a mounting fixture 25, it is possible to connect the urine drainage tube 13 to the urine tank 30 or the urine pack 31 by fixing the urine drainage tube 13 to the tube guide hole while maintaining the airtightness of the urine tank 30 or the urine pack 31. Therefore, replacement of the urine drainage tube can be carried out easily as well as cleanly.

As described above, in the present invention, a urine drainage tube can be replaced easily and cleanly since a mounting fixture, with a urine drainage tube inserted, fixes the urine drainage tube to a tube guide hole while maintaining the airtightness of a urine tank or a urine pack and connects the urine drainage tube to the urine tank or the urine pack.

Note that, although a one-touch joint is used on the urine drainage tubes that connect the urine receptacle and the urine tank in the embodiments of the present invention as described above, it is clear that the use of a one-touch joint can be omitted. Also, it is possible to use an IC chip as a urine sensor so that a urine detection signal can be transmitted wirelessly.

In the present invention, a urine drainage tube can be replaced easily and cleanly since a mounting fixture, with a urine drainage tube inserted, fixes the urine drainage tube to a tube guide hole while maintaining the airtightness of a urine tank or a urine pack and connects the urine drainage tube to the urine tank or the urine pack.

The preferred embodiment described herein are therefore illustrative and not restrictive, the scope of the invention being indicated by the appended claims and all variations which come within the meaning of the claims are intended to be embraced therein.

## Claims

1. An automatic urine disposal device having a urine receptacle (1) for absorbing urine discharged by a device wearer, a urine storage member (31) with tube guide holes (31a, 31b) for storing the urine collected by the urine receptacle, and a urine drainage tube (12, 13) for directing the urine from the urine receptacle to the urine storage member, wherein
a vacuum pump (51) for lowering pressure inside said urine storage member is provided, and
a mounting fixture (16, 18) to be freely attached to/detached from the tube guide hole (31a, 31b) of said urine storage member is provided with an insertion hole where said urine drainage tube (13) is inserted, **characterized in that** said mounting fixture (16, 18), along with said urine drainage tube, maintains the airthightness of said urine storage member.

2. The automatic urine disposal device according to Claim 1, **characterized in that**
said mounting fixture (16, 18) has a funnel-like shape and is made of a soft material, and
said urine storage member has a urine storage container (31) housed inside a urine tank (30).

3. The automatic urine disposal device according to Claim 1 or 2, **characterized in that**
said mounting fixture (16, 18) has a funnel-like shape and is formed by a soft material, and
said urine storage member (31) has the urine tank, a lid (35) with a tube guide hole (35a, 35b) for sealing the urine tank, and a urine bag (34) held inside the urine tank.

4. The automatic urine disposal device according to Claim 2, **characterized in that**
a lid case (40) for containing said vacuum pump (51) is placed on top of said urine tank, and
a cutout section for guiding said urine drainage tube into said tube guide hole is formed on a lid plate (41) of the lid case (40).

5. The automatic urine disposal device according to any of the proceeding Claims **characterized in that**
a second tube guide hole (31b) is formed on said urine storage member, one of the ends of a suction tube (14) for suctioning and drawing air from said urine storage member into said vacuum pump,
an insertion hole is formed for inserting and holding the other end of the suction tube, and
a funnel-shaped second mounting fixture (18) detachable from/attachable to said second tube guide hole is provided.

6. The automatic urine disposal device according to any of the proceeding Claims, **characterized in that**
a urine sensor for detecting urination into said urine receptacle and activating said vacuum pump is provided.

7. The automatic urine disposal device according to any of Claims 3-6, **characterized in that**
an elastically supported support plate (32) is provided on the bottom plate of said urine tank,
said urine bag (34) is placed on the support plate (32), and
a thread (34a) for opening and closing the urine bag is provided at the bag opening.

8. The automatic urine disposal device according to any of claims 3-7, **characterized in that**
said urine bag contains superabsorbent polymers that absorb urine and become gelatinized.

9. The automatic urine disposal device according to Claim 2, **characterized in that**
said urine storage container is a paper pack made of heavy paper.

10. The automatic urine disposal device according to any of claims 3-9, **characterized in that**
a partition plate 36 is installed inside said urine tank for separating the space for placing said urine bag (34) from the space for said suction tube to suction air.

11. An automatic urine disposal device, comprising
a urine receiver for suctioning urine discharged by a device wearer,
an elastically supported support plate (32) on the bottom plate of a urine tank,
a urine pack (31) made of heavy paper placed inside said urine tank (30) on said support plate (32), the urine pack having two tube guide holes (31a, 31b) on its top surface,
a urine drainage tube (12, 13) for directing urine from a urine drainage port formed on said urine receptacle into said urine pack (31) through said tube guide hole,
a vacuum pump (51) for suctioning air inside said urine tank by way of a suction tube,
a lid case (40) placed an the top of said urine tank (30) for housing said vacuum pump,
a first funnel-like mounting fixture (16) for fixing said urine drainage tube (13) in one of said tube guide holes (31a, 31b) while maintaining the airtightness of said urine tank, the first mounting fixture having an insertion hold for inserting and holding said urine drainage tube and being detachable from/attachable to said tube guide hole, and
a second funnel-like mounting fixture (18) for fixing said suction tube (14) in the other tube guide hole while maintaining the airtightness of said urine tank (30), the first mounting fixture having an insertion hold for inserting and holding said suction tube and being detachable from/attachable to said tube guide hole.
